Europäisches Patentamt

European Patent Office

Office européen des brevets

(19)

(11) **EP 0 939 862 B1**

(12) **FASCICULE DE BREVET EUROPEEN**

(45) Date de publication et mention
de la délivrance du brevet:
**13.02.2002 Bulletin 2002/07**

(21) Numéro de dépôt: **97947111.7**

(22) Date de dépôt: **20.11.1997**

(51) Int Cl.$^7$: **F15C 5/00**, A61M 37/00

(86) Numéro de dépôt international:
**PCT/FR97/02101**

(87) Numéro de publication internationale:
**WO 98/22719 (28.05.1998 Gazette 1998/21)**

(54) **VANNE MINIATURE POUR LE REMPLISSAGE DU RESERVOIR D'UN APPAREIL D'ADMINISTRATION TRANSDERMIQUE DE MEDICAMENT**

MIKROVENTIL ZUM FÜLLEN DES BEHÄLTERS EINES TRANSDERMALEN MEDIKAMENTENVERABREICHUNGSSYSTEMS

MINIATURE VALVE FOR FILLING THE RESERVOIR OF AN APPARATUS FOR THE TRANSDERMAL ADMINISTRATION OF MEDICINE

(84) Etats contractants désignés:
**AT BE CH DE DK ES FI FR GB GR IE IT LI LU MC NL PT SE**

(30) Priorité: **21.11.1996  FR 9614230**
 **29.05.1997  FR 9706613**

(43) Date de publication de la demande:
**08.09.1999  Bulletin 1999/36**

(73) Titulaire: **LABORATOIRES D'HYGIENE ET DE DIETETIQUE**
**21300 Chenove (FR)**

(72) Inventeurs:
• **ROSSI, Carole-Résidence Hamamélis**
 **F-31520 Ramonville (FR)**
• **MILLOT, Philippe**
 **F-21490 Orgeux (FR)**
• **ESTEVE, Daniel**
 **F-31520 Ramonville-Saint-Anne (FR)**
• **MIKLER, Claude**
 **F-21000 Dijon (FR)**
• **TEILLAUD, Eric**
 **F-21240 Talant (FR)**

(74) Mandataire: **Colas, Jean-Pierre et al Cabinet JP Colas 37, avenue Franklin D. Roosevelt**
**75008 Paris (FR)**

(56) Documents cités:
**EP-A- 0 513 879      WO-A-94/21314**
**US-A- 3 184 097      US-A- 5 186 001**

## Description

La présente invention est relative à une vanne miniature, à un dispositif de remplissage d'un réservoir comprenant une telle vanne et à un procédé de fabrication de ladite vanne. La présente invention est aussi relative à un appareil d'administration transdermique de médicament comprenant un tel dispositif de remplissage d'un réservoir avec une solution d'un principe actif à administrer.

De manière générale, pour administrer un médicament par voie transdermique, on applique un réservoir contenant un principe actif contre la peau du patient, ce réservoir étant couramment constitué par une couche d'un produit hydrophile tel qu'un hydrogel, portée par un support convenable. Quand l'administration de médicament est assistée par ionophorèse, le principe actif est en solution ionique. La solution ionique est conservée dans une poche que l'on vide dans le réservoir, immédiatement avant l'administration. Cette procédure est rendue nécessaire par le fait que la solution perd sa stabilité dans l'hydrogel constituant le réservoir et ne peut donc être chargée dans celui-ci ab initio.

Se pose alors le problème du vidage de la poche de solution ionique de principe actif dans le réservoir. Pour ce faire on a conçu divers moyens mécaniques tels que, par exemple, un moyen du type seringue dans lequel l'actionnement d'un piston chasse la solution ionique de la seringue pour l'injecter dans le réservoir. On a conçu aussi des poches souples à ouverture par piqûre ou déchirure et vidage dans le réservoir par l'effet d'une pression exercée sur la poche.

Il apparaît ainsi que, quels que soient les moyens employés pour vider la poche de solution ionique dans le réservoir, ces moyens exigent une intervention humaine, ce qui est peu pratique, et ne se prêtent pas à un déclenchement automatique, immédiatement avant le début d'un traitement. Par ailleurs, les moyens mécaniques évoqués ci-dessus sont sujets à des déficiences en matière d'étanchéité et n'assurent pas toujours un vidage complet de la poche dans le réservoir.

On sait par ailleurs que l'administration transdermique de médicament assistée par ionophorèse fait souvent appel à un matériel qui prend la forme d'un bracelet porté par un patient, ce bracelet portant tous les organes nécessaires à cette administration, de manière à assurer l'autonomie du patient. Le bracelet porte ainsi, outre le réservoir appliqué sur la peau de ce dernier, des électrodes, des moyens électroniques de commande de ces électrodes et une pile d'alimentation électrique de ces moyens, contenant donc une quantité d'énergie électrique limitée. Il serait souhaitable que le vidage, évoqué plus haut, de la solution ionique dans le réservoir, ou "hydratation" du réservoir, soit commandé par les moyens électroniques présents dans le bracelet de manière à automatiser ce vidage préalablement à un traitement, sans que cette fonction supplémentaire de ces moyens n'entraîne une consommation d'énergie électrique que la pile ne pourrait supporter.

La présente invention a précisément pour but de réaliser une vanne miniature propre à assurer ladite "hydratation" du réservoir de manière automatique, sous la commande desdits moyens électroniques, et ce pour une dépense d'énergie électrique aussi minime que possible, non susceptible d'altérer le fonctionnement du matériel portatif utilisé pour une administration transdermique assistée par ionophorèse.

La présente invention a aussi pour but de réaliser une telle vanne qui permette d'éliminer les problèmes d'étanchéité et de vidage incomplet de la poche, qui affectent parfois les moyens mécaniques d'hydratation de réservoir utilisés dans la technique antérieure.

La présente invention a encore pour but de réaliser une telle vanne qui puisse être fabriquée industriellement avec un taux de malfaçon très faible, voire nul.

On atteint ces buts de l'invention, ainsi que d'autres qui apparaîtront à la lecture de la description qui va suivre, avec une vanne miniature comprenant a) un substrat, b) une charge d'un matériau combustible disposé sur le substrat en regard d'un passage à ouvrir à travers celui-ci, c) une résistance électrique placée au contact de la charge combustible de manière que l'alimentation de cette résistance avec une énergie électrique prédéterminée assure la combustion de la charge et l'ouverture du passage, par rupture locale dudit substrat sous la pression des gaz de combustion de la charge.

Comme on le verra dans la suite, en plaçant une pluralité de telles vannes entre une poche d'une solution de principe actif et un réservoir à charger avec cette solution, on dispose de moyens permettant un déclenchement automatique de l'hydratation du réservoir, immédiatement avant un traitement, sans aucune intervention humaine, par une simple commande électronique de l'ouverture de ces vannes.

Suivant d'autres caractéristiques de la présente invention, la résistance est filiforme, la charge s'étend sur et au-delà de la résistance, de manière que la chaleur dégagée par la résistance alimentée par une énergie électrique prédéterminée se concentre initialement dans la fraction de la charge qui entoure la résistance. Ladite énergie électrique prédéterminée est inférieure à 10 Joules. Une dépense d'énergie aussi faible ne draine pas sensiblement la charge de la pile d'alimentation d'un appareil portatif d'application transdermique de médicament assistée par ionophorèse, et est donc compatible avec les autres prélèvements d'énergie à opérer sur cette pile.

Suivant encore une autre caractéristique de la présente invention, la charge et la résistance sont disposées d'un même côté d'une zone amincie du substrat, l'épaisseur et la surface de cette zone étant choisies pour que la pression des gaz due à la combustion de la charge provoque la fragmentation de cette zone et son ouverture au passage desdits gaz et d'autres fluides éventuels.

L'invention permet ainsi de réaliser un dispositif de remplissage d'un réservoir avec un fluide contenu dans une poche adjacente à ce réservoir, comprenant au moins une vanne miniature suivant l'invention, disposée de manière à obturer une communication de fluide prévue entre la poche et le réservoir, et des moyens pour commander sélectivement l'alimentation de la résistance électrique de la vanne et provoquer ainsi l'ouverture de ladite communication au fluide contenu dans la poche, à travers le passage ouvert dans le substrat de la vanne par la combustion de la charge qu'elle porte.

Suivant une autre caractéristique de ce dispositif celui-ci comprend en outre au moins une enveloppe souple intérieure à la poche, cette enveloppe enfermant une vanne miniature suivant la présente invention, les moyens de commande déclenchant sélectivement la combustion de la charge portée par cette vanne de manière à gonfler ladite enveloppe avec le gaz de combustion, pour assister ainsi le vidage de la poche à travers la ou les vannes disposées dans les communications de fluide entre ladite poche et ledit réservoir.

L'invention fournit aussi un procédé de fabrication de la vanne suivant l'invention, suivant lequel :

a) on recouvre une face sensiblement plane d'un substrat en matériau semi-conducteur avec une couche d'arrêt d'une solution de gravure dudit matériau,

b) on recouvre cette couche d'arrêt avec au moins une couche d'isolation électrique, quand la couche d'arrêt n'est pas électriquement isolante,

c) on forme sur ladite couche d'isolation électrique une résistance électrique,

d) on creuse par gravure le substrat, à partir de la face du substrat opposée à celle qui porte ladite résistance, jusqu'à la couche d'arrêt, et

e) on dépose une charge combustible par-dessus la résistance électrique.

D'autres caractéristiques et avantages de la présente invention apparaîtront à la lecture de la description qui va suivre et à l'examen du dessin annexé dans lequel :

- la figure 1 est une vue perspective schématique d'une vanne miniature selon la présente invention,
- les figures 2 et 3 sont des vues schématiques en coupe de la vanne de la figure 1, utiles à l'explication du fonctionnement de celle-ci,
- les figures 4 et 5 illustrent une mise en oeuvre particulière de la vanne miniature selon l'invention, dans une enveloppe gonflable,
- les figures 6 et 7 illustrent schématiquement la structure et le fonctionnement d'un dispositif de remplissage d'un réservoir d'une solution de principe actif, en vue d'une application transdermique de ce principe actif, comprenant des vannes miniatures suivant l'invention,

- les figures 8A à 8E illustrent les étapes successives d'un procédé de fabrication de la vanne miniature selon l'invention,
- les figures 9A à 9E illustrent les étapes successives d'une variante du procédé illustré aux figures 8A à 8E.

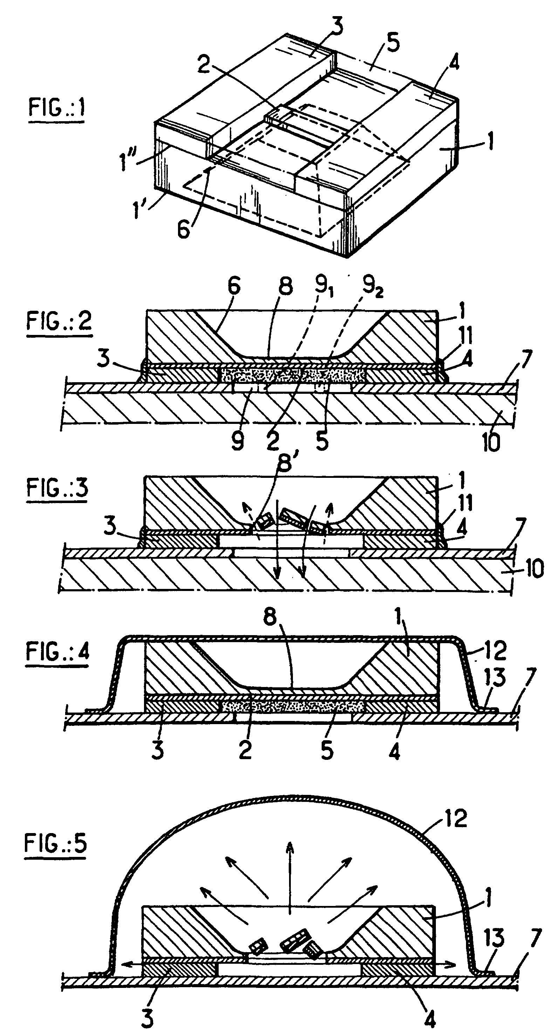

On se réfère à la figure 1 du dessin annexé où il apparaît que la vanne miniature selon l'invention prend, à titre d'exemple illustratif et non limitatif, une forme parallélépipèdique. Elle est constituée d'un substrat 1 portant une résistance électrique 2 dont les extrémités sont connectées à des contacts métalliques 3,4, la résistance 2 étant recouverte d'une charge 5 d'un matériau combustible en couche mince, représenté transparent pour la clarté de la figure, entre les contacts 3,4.

Ce matériau doit, pendant sa combustion, former des gaz de combustion nécessaires au fonctionnement de la vanne, comme on le verra plus loin. Toujours à titre d'exemple illustratif et non limitatif, on peut choisir à cet effet un matériau pyrotechnique du type nitrocellulose ou propergol par exemple. On a utilisé avec succès la nitrocellulose de type GB, et plus particulièrement, de type GBPA ainsi qu'un propergol à base de poly(azoture de glycidyle) commercialisés par la société française dite : Société Nationale des Poudres et Explosifs (S.N.P.E.). De telles nitrocelluloses et propergols peuvent être enflammées avec un minimum d'énergie thermique, ce qui les rend particulièrement appropriées à la présente invention, comme on le verra plus loin.

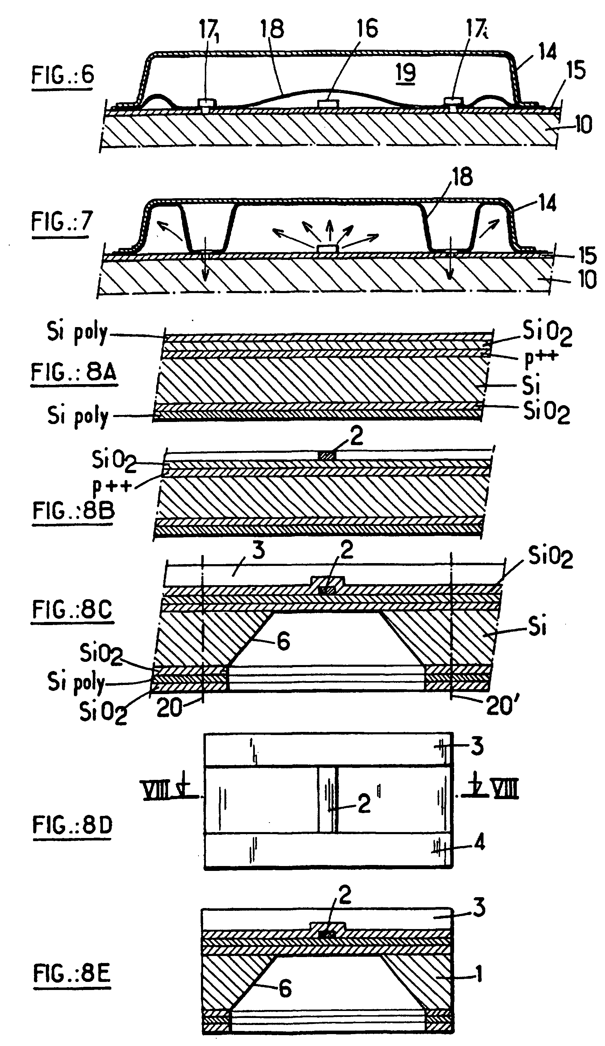

Le substrat 1 de la vanne de la figure 1 peut être réalisé avec un matériau semi-conducteur tel que le silicium, couramment utilisé dans les techniques de fabrication de circuits intégrés. La vanne suivant l'invention peut être fabriquée par ces techniques, comme on l'expliquera en liaison avec les figures 8A à 8E, ce qui permet de lui donner un grand degré de miniaturisation, celui d'une "puce" électronique par exemple, propre à permettre son intégration dans des appareils de faible encombrement tels que les appareils autonomes d'administration transdermique de médicament assistés par ionophorèse, décrits plus loin en liaison avec les figures 6 et 7.

Sur la figure 1 il apparaît encore que le substrat 1 de la vanne est creusé d'un cratère pyramidal tronqué 6, à partir de sa face 1', parallèle et opposée à celle (1") qui porte la résistance 2, le fond de ce cratère étant constitué par une membrane ou zone amincie 8 du substrat 1, accolée à cette résistance. Comme on le verra plus loin, l'épaisseur de cette zone amincie 8 doit être suffisamment faible pour que cette zone se rompt sous la pression des gaz dégagés par la combustion de la charge 5, déclenchée par chauffage à l'aide de la résistance 2 placée sur la zone amincie , du même côté que la charge 5.

On a représenté aux figures 2 et 3 la vanne ou "puce" de la figure 1, montée sur un circuit imprimé 7 propre à faire passer un courant électrique dans la résistance 2, entre les plots de contact 3 et 4 soudés à des pistes conductrices de ce circuit, d'une manière analogue à celle suivant laquelle les composants électroniques classiques sont montés sur de tels circuits. A l'endroit de la zone amincie 8 du substrat, le circuit 7 est percé d'un trou 9, ce trou étant lui-même obturé par une couche 10 d'un produit qui, dans l'application à l'administration transdermique de médicament envisagée plus haut peut être un hydrogel, ou un non-tissé, par exemple, à charger avec une solution d'un principe actif contenu dans une poche communiquant avec le cratère 6 du substrat. La charge 5 de matériau combustible se trouve ainsi disposée entre cette couche 10 et la zone amincie du substrat. Un joint 11 entoure le substrat 1 pour assurer, à la fois, la fixation de ce substrat sur le circuit 7 et l'étanchéité de l'espace dans lequel est contenue la charge 5.

On remarquera que la résistance 2 (voir figure 1) est de forme allongée ou filiforme et n'est ainsi adjacente qu'à une partie seulement de la charge 5 qui la recouvre.

Grâce au circuit imprimé 7, on peut déclencher automatiquement le passage d'un courant électrique dans la résistance 2, sous la commande d'un microprocesseur par exemple, utilisé classiquement par ailleurs pour commander l'exécution d'un programme d'administration d'un médicament.

Le passage du courant dans la résistance provoque, par effet Joule, un dégagement de chaleur qui, grâce à la conductivité thermique faible de la nitrocellulose constituant la charge 5, reste concentré dans cette charge autour de la résistance. Il s'ensuit un fort réchauffement local de la nitrocellulose, ajusté pour assurer l'inflammation de celle-ci. On remarquera qu'ainsi l'inflammation d'une fraction de la nitrocellulose seulement ne requiert qu'une faible quantité d'énergie électrique, ce qui est avantageux quand cette énergie provient d'une pile alimentant par ailleurs l'ensemble d'un appareil portatif d'administration transdermique de médicament assisté par ionophorèse. On réduit ainsi fortement le drainage d'énergie de la pile nécessaire à la mise à feu de la nitrocellulose, alors que ce produit est déjà bien adapté à l'application visée par l'invention, puisqu'il est susceptible de s'enflammer localement à de basses températures, avec propagation immédiate de la combustion dans tout le volume de la charge.

La combustion de la nitrocellulose ayant ainsi dégagé une quantité substantielle de gaz dans un faible volume (celui du trou 9), on peut régler la quantité de nitrocellulose brûlée pour que la pression dans ce trou atteigne une valeur suffisante pour provoquer la rupture et la dislocation (voir figure 3) de la zone amincie 8 du substrat adjacent au trou 9, cette zone amincie 8 étant alors remplacée par un passage 8' qui permet aux gaz formés de s'échapper, comme schématisé par les flèches en traits interrompus. Il permet aussi à un liquide mis en communication avec le cratère 6, de passer à travers le passage 8' et le trou 9, suivant la flèche en trait plein, pour venir ainsi imbiber la couche d'hydrogel 10 constituant un réservoir d'appareil d'administration transdermique de médicament, par exemple.

Le large trou 9 percé dans le circuit 7 peut être remplacé par une pluralité de trous décentrés plus petits $9_1, 9_2$, etc... représentés en trait interrompu à la figure 2, de surface cumulée inférieure à celle du trou 9. On peut suppléer ainsi à une éventuelle fragilité de la couche 10, pour confiner les gaz issus de la combustion de la charge 5.

On a pu réaliser une vanne miniature selon l'invention, sur un substrat de 3mm x 3mm, portant une charge de $8.10^{-4}$ g de nitrocellulose environ, formant par combustion 8 ml de gaz à une pression suffisante pour détruire une zone amincie 8 de 3 à 5 $\mu$m d'épaisseur environ, après le passage dans la résistance d'un courant électrique d'une puissance de 1 W pendant 1 seconde.

Il apparaît ainsi que l'invention permet bien d'atteindre les buts annoncés, à savoir fournir une vanne miniature, prenant la forme d'une puce électronique, pouvant être déclenchée automatiquement à l'aide d'un signal électrique de faible puissance, compatible avec celle que peut délivrer une pile électrique d'alimentation d'un appareil électronique portatif, tel qu'un appareil d'administration transdermique de médicament assistée par ionophorèse.

On se réfère maintenant aux figures 4 et 5 où on a représenté un agencement particulier de la vanne suivant l'invention, faisant fonction alors de générateur de gaz, utilisable pour forcer un fluide hors d'une poche, cet agencement étant utilisable en particulier dans le dispositif de remplissage de réservoir représenté aux figures 6 et 7 du dessin annexé, comme on le verra plus loin.

A la figure 4 on retrouve la vanne des figures 2 et 3, montée de même sur un circuit imprimé 7. Cette fois-ci, cependant, la charge 5 n'est pas disposée en regard d'un trou du circuit et cette charge 5 est donc confinée entre la zone amincie 8 du substrat et la surface adjacente du circuit 7, sur laquelle la vanne est fixée par tout moyen convenable, soudure, colle, etc... Une enveloppe gonflable 12, une feuille souple de matière plastique, par exemple, soudée sur le circuit 7 à la périphérie de la vanne, enferme celle-ci dans l'espace étanche intérieur à cette enveloppe.

Tout comme pour la vanne des figures 2 et 3, le passage d'un courant dans la résistance 2 provoque par effet Joule l'échauffement local de la charge 5 qui s'enflamme en dégageant une quantité de gaz sous pression calculée pour gonfler cette enveloppe, comme représenté à la figure 5. on remarquera l'absence du joint d'étanchéité 11 de la vanne des figures 2 et 3, les gaz dégagés par la combustion pouvant s'échapper de la vanne de tous côtés. L'objectif est d'ici de gonfler l'enveloppe étanche 12, disposition dont l'intérêt apparaîtra lors de la description qui va suivre du dispositif représenté aux figures 6 et 7, conçu pour assurer sélectivement le remplissage automatique d'un réservoir 10 avec un liquide contenu dans une poche 14 adjacente au réservoir.

A titre d'exemple illustratif et non limitatif, le dispositif des figures 6 et 7 fait partie d'un appareil d'administration transdermique de médicament assisté par ionophorèse. Comme on l'a vu plus haut en préambule de la présente description, un tel appareil comprend couramment un ensemble d'électrodes et au moins un réservoir tel que 10 conçu pour s'appliquer sur la peau d'un patient. Une électrode (non représentée) est accolée au réservoir 10 et coopère avec une contre-électrode (non représentée) pour forcer des ions d'un principe actif contenus dans le réservoir 10, à descendre un champ électrique établi entre les électrodes, ces ions passant ainsi sous la peau du patient. L'appareil est portatif et comprend en outre des moyens électroniques de commande du champ établi entre les électrodes et une pile électrique pour alimenter ces moyens et les électrodes. Tout ceci est bien connu de l'homme de métier et n'exige pas une description plus complète.

La vanne suivant l'invention permet de réaliser un tel dispositif pour lequel le chargement ou "l'hydratation" du réservoir 10, une couche d'hydrogel par exemple, avec une solution ionique de principe actif contenue dans une poche 14 adjacente au réservoir 10, est déclenché automatiquement par les moyens électroniques de commande de l'appareil, avant le commencement du traitement.

Pour ce faire la poche 14 et le réservoir 10 sont fixés sur deux faces opposées d'une feuille 15, souple, susceptible de porter un circuit d'alimentation de vannes suivant l'invention. Une première vanne 16 est fixée sur la feuille 15, comme la vanne des figures 4 et 5, dans une position centrale, par exemple, à l'intérieur de la poche 14, alors qu'une pluralité d'autres vannes $17_1, 17_2, ...$ $17_i$, etc... sont montées sur la même feuille autour de la vanne 16, comme la vanne des figures 2 et 3. Une enveloppe souple 18 est soudée à la feuille 15 et à la poche 14 au niveau de leur périphérie commune (voir les figures 6 et 7), les vannes $17_i$ pinçant en outre l'enveloppe 18 contre la feuille 15, là où elles sont fixées sur cette feuille.

Cette dernière porte des pistes conductrices (non représentées) d'alimentation des contacts 3,4 des vannes 16 et $17_i$, le passage d'un courant entre ces contacts étant sélectivement commandé par les moyens électroniques évoqués ci-dessus.

On comprend que, lorsque ces moyens déclenchent la mise à feu des charges combustibles contenues dans ces vannes, les vannes $17_i$ ouvrent chacune une communication de fluide, ou passage, entre la poche 14 et le réservoir 10, à travers la zone amincie éclatée d'une vanne et un trou coaxial percé dans la feuille 15, alors que les gaz dégagés par la combustion de la charge de la vanne 16 provoque le gonflement de l'enveloppe 18 (voir figure 7). Celle-ci vient occuper une grande partie, voir la totalité du volume intérieur à la poche 14, en chassant la solution ionique 19 contenue dans celle-ci, cette solution étant ainsi forcée dans le réservoir 10 à travers les vannes $17_i$ et les trous coaxiaux percés dans la feuille 10, les vannes $17_i$ ayant été préalablement ouvertes par des commandes électriques appropriées émises par les moyens électroniques.

Ainsi ces moyens, qui commandent l'exécution d'un programme d'administration de médicament après "l'hydratation" du réservoir, déclenchent-ils aussi cette hydratation, préalablement au traitement, sans qu'aucune intervention humaine telle qu'actionnement de seringue, déchirure d'une enveloppe frangible, etc,... ne soit nécessaire pour assurer l'hydratation. La fiabilité de cette opération en est renforcée. L'étanchéité du matériel utilisé est améliorée du fait qu'aucun perçage ou déchirure d'une enveloppe souple n'est nécessaire pour provoquer l'hydratation. Le vidage de la poche 14 peut être ajusté par un gonflement précis de l'enveloppe 18, ce qui assure un chargement de l'hydrogel du réservoir avec une quantité bien définie de la solution ionique, assurant la reproductibilité de ce chargement d'un ensemble d'électrodes à un autre.

On se réfère maintenant aux figures 8A à 8E du dessin annexé pour décrire un procédé de fabrication de la vanne miniature selon l'invention. Celui-ci fait avantageusement appel à des technologies de formation de couches et de microgravure bien connues dans la fabrication de circuits intégrés, ce qui permet d'assurer une fabrication à bas prix de ces vannes, et une bonne reproductibilité de leurs performances.

On part d'une plaquette de silicium (voir figure 8A) dont on dope fortement une face pour lui donner une conductivité de type $P^{++}$, sur 2 μm environ d'épaisseur, par exemple. Les deux faces de la plaquette sont ensuite isolées électriquement avec des couches de dioxyde de silicium ($SiO_2$) de 0,5 μm, elles-mêmes recouvertes de couches de silicium polycristallin (Si poly) de 0,5 μm également, dopées N. La couche de silicium polycristallin qui est du même côté du substrat de silicium Si que la couche dopée $P^{++}$ est ensuite gravée par plasma pour délimiter la résistance filiforme 2 (vue en coupe transversale à la figure 8B).

La résistance est ensuite protégée par une oxydation en surface du silicium polycristallin, qui forme alors une couche superficielle de $SiO_2$ qui se raccorde, au-delà de la résistance 2, à la couche de $SiO_2$ sous-jacente (voir figure 8C). Des contacts à l'or 3,4 sont formés aux extrémités de la résistance 2 par un procédé de métallisation classique.

Après gravure par plasma d'un masque dans les couches de $SiO_2$ formées sur l'autre face de la plaquette, et gravure du cratère 6 dans le silicium sous-jacent jusqu'à la couche d'arrêt de gravure constituée par la couche $P^{++}$, on découpe la plaquette de silicium suivant les traits de coupe 20,20' pour individualiser la puce représentée au figures 8D et 8E, en plan et en coupe suivant le trait VIII-VIII respectivement. Il reste à déposer au-dessus de la résistance 2, et entre les contacts 3,4, une goutte de nitrocellulose, ou à coller entre ces contacts un élément d'une feuille de nitrocellulose, pour compléter une vanne miniature suivant l'invention avec sa charge combustible.

En variante, la couche dopée P$^{++}$ et la couche de SiO$_2$ qui la recouvre (voir figure 8A) peuvent être remplacées par une couche unique de SiO$_2$ prenant la forme d'une membrane de très faible épaisseur. Celle-ci joue alors à la fois le rôle de couche d'arrêt de gravure et de couche d'isolation électrique pour la résistance 2.

On a cependant rencontré des difficultés avec une telle membrane en silice. On a pu observer l'existence, dans une telle membrane, d'une contrainte de compression supérieure à 0,1 GPa. Une telle contrainte peut provoquer une forte déformation de la membrane. A titre d'exemple, on a pu observer et mesurer une flèche de 40 $\mu$m sur une membrane de 1 $\mu$m d'épaisseur. Une telle déformation peut provoquer une rupture de la membrane, génératrice de malfaçon dans une fabrication industrielle de masse.

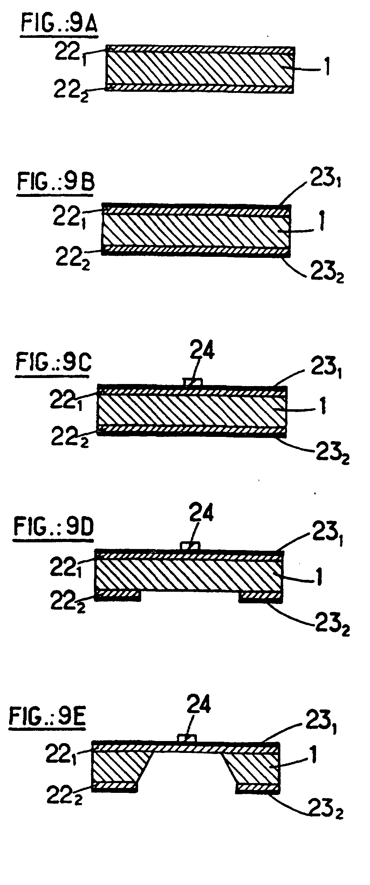

On se réfère aux figures 9A à 9E du dessin annexé pour décrire une variante du procédé de fabrication décrit ci-dessus en liaison avec les figures 8A à 8E, cette variante étant propre à permettre une fabrication industrielle de la vanne miniature suivant l'invention, avec un taux de malfaçon très faible, voire nul.

On part d'une puce de silicium, généralement plane, constituant le substrat 1. On forme sur chacune des deux faces opposées de la puce une couche de silice 22$_1$,22$_2$ respectivement (voir figure 9A), par exemple par oxydation thermique du silicium à 1150° C en atmosphère humide. L'épaisseur de la couche de silice est typiquement comprise entre 0,5 et 1,5 $\mu$m.

Comme on l'a vu plus haut, on observe dans une telle couche de silice déposée sur un substrat de silicium une contrainte de compression (de l'ordre de 0,27 GPa) susceptible de déformer et de briser la couche, là où celle-ci est privée de son support de silicium comme c'est le cas dans la zone amincie 8 (voir figure 2).

Suivant la présente invention, on compense sensiblement les effets de l'existence de cette contrainte de compression en couvrant la couche de silice avec une couche de nitrure de silicium dans laquelle on observe la présence d'une contrainte de tension. En combinant les effets de ces contraintes contraires, on peut abaisser la contrainte résiduelle s'exerçant dans la zone amincie 8, jusqu'à un niveau de contrainte non susceptible de déformer ou de rompre cette zone pendant la fabrication de la vanne miniature, ce niveau étant typiquement inférieur à ± 0,1 GPa, les signes + et - étant attachés à des contraintes de tension et de compression respectivement. Ce résultat peut être obtenu en ajustant convenablement les épaisseurs des deux couches à l'aide de la relation :

$$\sigma_r = \frac{e_{ox} \cdot \sigma_{ox} + e_{nit} \cdot \sigma_{nit}}{e_{ox} + e_{nit}}$$

où $\sigma_r$, $\sigma_{ox}$, $\sigma_{nit}$ sont, respectivement, la contrainte résiduelle dans la zone amincie, la contrainte compression dans la couche de silice et la contrainte de tension dans la couche de nitrure de silicium,

et $e_{ox}$, $e_{nit}$ les épaisseurs respectives des couches de silice et de nitrure de silicium.

Les contraintes mentionnées ci-dessus peuvent être mesurées à partir des déformations subies par une plaquette de silicium de 7,5 x 7,5 cm du fait du dépôt sur celle-ci d'une des couches mises en jeu.

A l'étape du procédé selon l'invention illustrée à la figure 9B, on forme donc sur les couches 22$_1$,22$_2$ de silice, des couches 23$_1$,23$_2$ respectivement de nitrure de silicium.

Celui-ci peut être du nitrure de silicium stoechiométrique Si$_3$N$_4$. La couche de Si$_3$N$_4$ peut être formée par dépôt en phase vapeur à basse pression à 750°C environ à partir de dichlorosilane SiH$_2$Cl$_2$ et d'ammoniac. On a relevé dans une telle couche des contraintes de tension de l'ordre de 1,2 GPa propres à compenser la contrainte de compression régnant dans la couche de silice adjacente. Cependant, le niveau élevé de cette contrainte est responsable d'une adhérence médiocre de la couche de Si$_3$N$_4$ sur la couche de silice, qui affecte défavorablement le taux de malfaçon observé sur une fabrication de masse.

Suivant la présente invention, on améliore considérablement le taux de malfaçon observé en remplacement la couche de nitrure de silicium stoechiométrique par une couche de nitrure de silicium SiN$_x$ dopée en silicium, x étant inférieur à 1,33. La contrainte de tension (0,6 GPa) observée dans une telle couche est plus faible que celle observée dans le nitrure de silicium stoechiométrique, ce qui permet d'éliminer le problème d'adhérence évoqué ci-dessus et de porter le taux de malfaçon à un niveau très faible, voire nul.

La formation d'une telle couche de nitrure de silicium enrichi en silicium peut être obtenue par dépôt en phase vapeur à basse pression, à 750°C environ, à partir d'hydrure de silicium SiH$_4$ et d'ammoniac.

Suivant un mode de réalisation préféré de la présente invention, on choisi x = 1,2 environ. La composition de SiN$_{1,2}$ peut être déduite d'une mesure de l'indice de réfraction de la couche par ellipsométrie, à 830 nm.

Après avoir ainsi déposé sur chacune des faces du substrat une couche de silice et une couche de nitrure de silicium, préférablement enrichi en silicium, présentant des épaisseurs prédéterminées à la lumière des considérations présentées ci-dessus, le procédé de fabrication se poursuit par la formation, sur l'une des faces du substrat, d'une résistance filiforme 24 (voir figure 9C) réalisée classiquement par dépôt d'une couche de silicium polycristallin, puis gravure de cette couche pour délimiter ladite résistance. Celle-ci présente typiquement une section droite de 0,5 x 100 µm et une longueur de 1,5 mm. Des contacts métalliques (non représentés) sont formés aux extrémités de la résistance pour permettre l'alimentation électrique de celle-ci, tout comme les contacts 3,4 de la vanne représentée à la figure 2. La résistance 24 ainsi formée est bien isolée par son support, la couche de nitrure de silicium, qui est un matériau diélectrique.

On forme ensuite sur l'autre face du substrat une fenêtre 25 dans les couches $22_2$ et $23_2$, par le procédé classique de masquage et de gravure au plasma de $CF_4$ gazeux. Un cratère 6 analogue à celui représenté à la figure 2 est ensuite creusé à travers la fenêtre 25 à l'aide d'un produit de gravure anisotropique approprié, tel qu'un hydroxyde de tétraméthylammonium, voir figure 9D. La gravure est arrêtée par la couche de silice $22_1$. La résistance 24 est alors portée par une zone amincie, ou membrane, bi-couche suivant la présente invention (voir figure 9E).

La vanne miniature est ensuite complétée par le dépôt d'une charge de matériau combustible sur la résistance 24 puis montée sur un circuit imprimé, tout comme la vanne représentée à la figure 2. A titre d'exemple illustratif et non limitatif, cette charge peut être constituée par un propergol à base de poly(azoture de glycidyle).

On a réalisé des vannes à membrane bi-couche suivant l'invention selon trois configurations différentes, à savoir :

1) une couche de silice de 1 µm d'épaisseur, associée à une couche de $Si_3N_4$ de 0,22 µm ;
2) une couche de silice de 0,5 µm d'épaisseur associée à une couche de $SiN_{1,2}$ de 0,22 µm ;
3) une couche de silice de 1,4 µm d'épaisseur associée, à une couche de $SiN_{1,2}$ de 0,6 µm ;

Si le taux de malfaçon observé avec la configuration 1 était encore substantiel, ce taux est tombé à 5 % et 0 % avec les configurations 2) et 3), respectivement.

Il apparaît ainsi que l'association d'une couche de silice et d'une couche de $SiN_{1,2}$ permet bien d'atteindre un but essentiel de la présente invention, à savoir une production de vannes miniatures comportant une résistance miniature portée par une membrane de très faible épaisseur, par exemple comprise entre 0,7 et 2 µm, la fabrication de cette membrane pouvant être pratiquée avec un taux de malfaçon très faible, voire nul.

Par ailleurs, les mesures opérées ont montré que les caractéristiques thermiques de la résistance ainsi montée sont très bonnes, celle-ci étant capable de faire monter la température de la charge combustible constituée du propergol précité à 300° avec une puissance électrique inférieure à 1 W appliquée pendant un temps inférieur à 200 ms, conformément à un autre but poursuivi par la présente invention.

Bien entendu, l'invention n'est pas limitée aux modes de réalisation décrits et représentés qui n'ont été donnés qu'à titre d'exemple. C'est ainsi que d'autres matériaux semi-conducteurs pourraient être utilisés en lieu et place du silicium pour constituer le substrat de la vanne, du germanium par exemple. De même des matériaux combustibles autres que la nitrocellulose pourrait constituer la charge combustible, d'autres matériaux "pyrotechniques" par exemple. Des techniques de fabrication autres que celles relevant de la fabrication de circuits intégrés pourraient aussi permettre de réaliser la vanne suivant l'invention.

Egalement, l'invention s'étend à des applications autres que le chargement d'un réservoir en solution ionique de médicament pour application transdermique assistée par ionophorèse. C'est ainsi que l'invention trouve aussi application au chargement d'un réservoir utilisé en administration transdermique passive classique. Elle s'étend plus généralement à toute application dans laquelle on doit provoquer une mise en communication de fluide sans accès mécanique à la vanne déclenchant cette mise en communication, par exemple dans des implants d'administration sous-cutanée, vasculaire ou musculaire par exemple, de médicaments.

**Revendications**

1. Vanne miniature, **caractérisée en ce qu'**elle comprend a) un substrat (1), b) une charge (5) d'un matériau combustible disposée sur le substrat en regard d'un passage à ouvrir à travers celui-ci, c) une résistance électrique (2) placée au contact de la charge combustible de manière que l'alimentation de cette résistance avec une énergie électrique prédéterminée assure la combustion de la charge (5) et l'ouverture du passage (8'), par rupture locale dudit substrat (1) sous la pression des gaz de combustion de la charge (5).

2. Vanne miniature conforme à la revendication 1, **caractérisée en ce que** la résistance (2) est filiforme, **en ce que** la charge (5) s'étend sur et au-delà de la résistance (2), de manière que la chaleur dégagée par la résistance alimentée par ladite énergie électrique prédéterminée se concentre initialement dans la fraction de la charge (5) qui entoure la résistance (2).

3. Vanne miniature conforme à la revendication 2, **caractérisée en ce que** ladite énergie électrique prédéterminée est inférieure à 10 Joules.

4. Vanne miniature conforme à l'une quelconque des revendications 1 à 3, **caractérisée en ce que** la charge (5) et la résistance (2) sont disposées d'un même côté d'une zone amincie (8) du substrat (1), l'épaisseur de cette zone étant choisie pour que la pression des gaz dus à la combustion de la charge (5) provoque la fragmentation de cette zone (8) et son ouverture au passage desdits gaz et d'autres fluides éventuels.

5. Vanne miniature conforme à la revendication 4, **caractérisée en ce que** ledit substrat (1) comprend deux faces (1', 1") sensiblement parallèles, l'une (1') étant creusée d'un cratère central (6) bouché, au niveau de l'autre face (1"), par ladite zone amincie (8).

6. Vanne miniature conforme à la revendication 5, **caractérisée en ce que** des contacts métalliques (3, 4) sont formés sur ladite autre face (1"), pour l'alimentation de la résistance (2) à partir d'une source d'énergie électrique extérieure.

7. Vanne conforme à l'une quelconque des revendications 1 à 6, **caractérisée en ce que** le substrat (1) prend la forme d'une puce en un matériau semi-conducteur, et **en ce que** ledit cratère (6) et ladite résistance (2) sont formés sur cette puce par microgravure.

8. Vanne conforme à l'une quelconque des revendications précédentes, **caractérisée en ce que** la charge combustible est constituée par un matériau du type nitrocellulose ou du type propergol.

9. Vanne conforme à la revendication 7, **caractérisée en ce que** le poids de la charge combustible est de l'ordre de $10^{-3}$ g.

10. Vanne conforme à la revendication 4, **caractérisée en ce que** ladite zone amincie (8) dudit substrat (1) qui porte ladite résistance électrique (2) est constituée au moins d'une première couche en silice et d'une deuxième couche, superposée à la première, en nitrure de silicium.

11. Vanne conforme à la revendication 10, **caractérisée en ce que** la deuxième couche est constituée de nitrure de silicium enrichi en silicium $SiN_x$ avec $x < 1,33$.

12. Vanne conforme à la revendication 11, **caractérisée en ce que** $x = 1,2$ environ.

13. Vanne conforme à la revendication 10, **caractérisée en ce que** les première et deuxième couches de $SiO_2$ et $Si_3N_4$ respectivement, présentent des épaisseurs d'environ 1µm et 0,22 µm, respectivement.

14. Vanne conforme à la revendication 12, **caractérisée en ce que** les première et deuxième couches de $SiO_2$ et $SiN_{1,2}$, respectivement, présentent des épaisseurs d'environ 0,5 µm et 0,22 µm, respectivement.

15. Vanne conforme à la revendication 12, **caractérisée en ce que** les première et deuxième couches de $SiO_2$ et $SiN_{1,2}$, respectivement, présentent des épaisseurs d'environ 1,4 µm et 0,6 µm, respectivement.

16. Dispositif de remplissage d'un réservoir (10) avec un fluide contenu dans une poche (14) adjacente à ce réservoir, **caractérisé en ce qu'**il comprend au moins une vanne miniature ($17_i$) conforme à l'une quelconque des revendications 1 à 15, disposée de manière à obturer une communication de fluide prévue entre la poche (14) et le réservoir (10), et des moyens pour commander sélectivement l'alimentation de la résistance électrique (2) de la vanne et provoquer ainsi l'ouverture de ladite communication au fluide contenu dans la poche (14), à travers le passage ouvert dans le substrat (1) de la vanne par la combustion de la charge (5) qu'elle porte.

17. Dispositif conforme à la revendication 16, **caractérisé en ce qu'**il comprend une pluralité de vannes miniatures ($17_i$) disposées dans autant de communications de fluide prévues entre la poche (14) et le réservoir (10), l'ouverture de chacune étant déclenchée par lesdits moyens de commande.

18. Dispositif conforme à l'une quelconque des revendication 16 et 17, **caractérisé en ce qu'**il comprend en outre au moins une enveloppe souple (18) intérieure à la poche (14), cette enveloppe (18) enfermant une vanne miniature (16) conforme à l'une quelconque des revendications 1 à 7, lesdits moyens de commande déclenchant sélectivement la combustion de la charge combustible portée par ladite vanne (16) de manière à gonfler ladite enveloppe (18) avec les gaz de combustion, pour assister ainsi la vidange de la poche (14) à travers la ou les vannes ($17_i$) disposées dans des communications de fluide entre ladite poche (14) et ledit réservoir (10).

19. Appareil d'administration transdermique de médicaments, du type dans lequel une solution de principe actif est initialement contenue dans une poche (14) puis transférée, avant le lancement d'un traitement, dans un réservoir (10) destiné à venir en contact avec la peau d'un patient, **caractérisé en ce que** ledit dispositif est conforme à l'une quelconque des revendications 16 à 18.

20. Procédé de fabrication d'une vanne conforme à l'une quelconque des revendications 1 à 15, **caractérisé par** les étapes suivantes :

a) on recouvre une face sensiblement plane d'un substrat (1) en matériau semi-conducteur avec une couche d'arrêt d'une solution de gravure dudit matériau ;

b) on recouvre cette couche d'arrêt avec au moins une couche d'isolation électrique, quand la couche d'arrêt n'est pas électriquement isolante ;

c) on forme sur ladite couche d'isolation électrique une résistance électrique ;

d) on creuse par gravure le substrat (1), à partir de la face (1") du substrat opposée à celle qui porte ladite résistance (2), jusqu'à la couche d'arrêt, et

e) on dépose une charge combustible (5) par dessus la résistance électrique (2).

21. Procédé conforme à la revendication 20, **caractérisé en ce qu'**on utilise du silicium pour constituer le substrat, du polysilicium dopé N pour constituer la couche conductrice, du dioxyde de silicium ($SiO_2$) pour constituer les couches d'isolation électrique.

22. Procédé conforme à la revendication 20, **caractérisé en ce que** ladite couche d'arrêt est une couche de silice électriquement isolante, **en ce qu'**on recouvre la couche de silice avec une couche de nitrure de silicium ($23_1$) et **en ce qu'**on forme ladite résistance électrique 24 sur ladite couche de nitrure de silicium ($23_1$).

23. Procédé conforme à la revendication 21, **caractérisé en ce qu'**on forme une couche ($23_1$) de nitrure de silicium stoechiométrique $Si_3N_4$ par dépôt en phase vapeur à basse pression, à partir de dichlorosilane et d'ammoniac, à 750°C environ.

24. Procédé conforme à la revendication 22, **caractérisé en ce qu'**on forme une couche ($23_1$) de nitrure de silicium $SiN_x$ enrichi en silicium, par dépôt en phase vapeur à basse pression, à partir d'hydrure de silicium et d'ammoniac, à 750°C environ.

## Claims

1. Miniature valve, **characterised in that** it comprises a) a substrate (1), b) a charge (5) of a combustible material disposed on top of the substrate facing a passage to be opened through the latter, c) an electrical resistance (2) in contact with the combustible charge so that supplying a predetermined electrical energy to this resistance assures the combustion of the charge (5) and the opening of the passage (8') by localised rupture of said substrate (1) due to the pressure of the gases of combustion of the charge (5).

2. Miniature valve according to claim 1, **characterised in that** the resistance (2) is filamentary and **in that** the charge (5) extends over and beyond the resistance (2) so that the heat generated by the resistance supplied with said predetermined electrical energy is initially concentrated in the portion of the charge (5) surrounding the resistance (2).

3. Miniature valve according to claim 2, **characterised in that** said predetermined electrical energy is less than 10 Joules.

4. Miniature valve according to any one of claims 1 to 3, **characterised in that** the charge (5) and the resistance (2) are disposed on the same side of a thinner area (8) of the substrate (1), the thickness of this area being such that the pressure of the gases due to the combustion of the charge (5) fragments this area (8) and opens it to the passage of said gases and possibly other fluids.

5. Miniature valve according to claim 4, **characterised in that** said substrate (1) has two substantially parallel faces (1', 1"), one face (1') having a central crater (6) recessed into it closed off at the other face (1") by said thinner area (8).

6. Miniature valve according to claim 5, **characterised in that** metal contacts (3, 4) are formed on said other face (1") for supplying energy to the resistance (2) from an external source of electrical energy.

7. Miniature valve according to any one of claims 1 to 6, **characterised in that** the substrate (1) takes the form of a semiconductor material chip and **in that** said crater (6) and said resistance (2) are formed on this chip by micro-etching.

8. Valve according to any one of the preceding claims **characterised in that** the combustible charge consists of a nitrocellulose or propergol type material.

9. Valve according to claim 7, **characterised in that** the weight of the combustible charge is in the order of $10^{-3}$ g.

10. Valve according to claim 4, **characterised in that** said thinner area (8) of said substrate (1) that carries said electrical resistance (2) comprises at least a first layer of silica and a second layer of silicon nitride on top of the first layer.

11. Valve according to claim 10, **characterised in that** the second layer is of silicon nitride enriched with silicon $SiN_x$ with $x < 1.33$.

12. Valve according to claim 11 **characterised in that** $x = 1.2$ approximately.

**13.** Valve according to claim 10, **characterised in that** the first and second layers of $SiO_2$ and $Si_3N_4$, respectively, have thicknesses of approximately 1 µm and 0.22 µm, respectively.

**14.** Valve according to claim 12, **characterised in that** the first and second layers of $SiO_2$ and $SiN_{1.2}$, respectively, have thicknesses of approximately 0.5 µm and 0.22 µm, respectively.

**15.** Valve according to claim 12, **characterised in that** the first and second layers of $SiO_2$ and $SiN_{1.2}$, respectively, have thicknesses of approximately 1.4 µm and 0.6 µm, respectively.

**16.** Device for filling a reservoir (10) with a fluid contained in a sachet (14) adjacent the reservoir, **characterised in that** it comprises at least one miniature valve ($17_i$) according to any one of claims 1 to 15 disposed in such manner as to obstruct fluid communication between this sachet (14) and the reservoir (10) and means for selectively commanding energisation of the electrical resistance (2) of the valve so as to cause opening of said communication to the fluid contained in the sachet (14) through the passage opened in the substrate (1) of the valve by the combustion of the charge (5) that it carries.

**17.** Device according to claim 16, **characterised in that** it comprises a plurality of miniature valves ($17_i$) disposed in the same number of fluid communications between the sachet (14) and the reservoir (10), the opening of each of the latter being initiated by said control means.

**18.** Device according to anyone of claims 16 and 17, **characterised in that** it further comprises at least one flexible envelope (18) inside the sachet (14), this envelope (18) enclosing a miniature valve (16) according to any one of claims 1 to 7, said control means selectively triggering the combustion of the combustible charge carried by said valve (16) so as to inflate said envelope (18) with the combustion gases to assist the emptying of the sachet (14) through the valve(s) ($17_i$) disposed in fluid communications between said sachet (14) and said reservoir (10).

**19.** Apparatus for transdermal administration of medication of the type in which an active principle solution is initially contained in a sachet (14) and then, prior to the commencement of a treatment, transferred into a reservoir (10) adapted to be placed in contact with the skin of a patient, **characterised in that** said reservoir (10) and said sachet (14) are parts of a device in accordance with any one of claims 16 to 18.

**20.** Method of manufacturing a valve according to any one of claims 1 to 15, **characterised by** the following steps:

a) a substantially plane face of a semiconductor material substrate (1) is covered with an etch stop layer resistant to a solution for etching said material ;
b) said etch stop layer is covered with at least an electrical insulation layer if the said stop layer is not electrically insulative ;
c) an electrical resistance is formed on said electrical insulation layer ;
d) the substrate (1) is etched from the face (1") of the substrate opposite that which carries said resistance (2), as far as said stop layer, and
e) a combustible charge (5) is deposited on top of the electrical resistance (2).

**21.** Method according to claim 20, **characterised in that** silicon is used for the support, N-doped polysilicon is used for the conductive layer and silicon dioxide ($SiO_2$) is used for the electrically insulative layers.

**22.** Method according to claim 20, **characterised in that** said stop layer is an electrically insulating silica layer, **in that** said silica layer is covered with a silicon nitride layer ($23_1$) and **in that** said electrical resistance (24) is formed onto said silicon nitride layer ($23_1$).

**23.** Method according to claim 21, **characterised in that** a layer ($23_1$) of stoicheometric silicon nitride $Si_3N_4$ is formed by low-pressure vapour phase deposition from dichlorosilane and ammonia at approximately 750°C.

**24.** Method according to claim 22, **characterised in that** a layer ($23_1$) of silicon nitride $SiN_x$ enriched with silicon is formed by low-pressure vapour phase deposition from silicon hydride and ammonia at approximately 750°C.

**Patentansprüche**

**1.** Miniaturventil, **dadurch gekennzeichnet, daß** es folgendes umfaßt: a) ein Substrat (1), b) eine Ladung (5) aus einem brennbaren Material, die auf dem Substrat gegenüber einem durch dieses hindurch zu öffnenden Durchgang angeordnet ist, c) einen elektrischen Widerstand (2), der im Kontakt mit der Brennstoffladung angeordnet ist, so daß die Versorgung dieses Widerstands mit einer vorgegebenen elektrischen Energie die Verbrennung der Ladung (5) und die Öffnung des Durchgangs (8') durch örtlichen Bruch des Substrats (1) unter dem Druck der Verbrennungsgase der Ladung (5) gewährleistet.

2. Miniaturventil nach Anspruch 1, **dadurch gekennzeichnet, daß** der Widerstand (2) fadenförmig ist, daß die Ladung (5) sich auf dem Widerstand (2) und über diesen hinaus erstreckt, so daß die Wärme, die von dem mit der vorgegebenen elektrischen Energie versorgten Widerstand abgegeben wird, sich anfangs in dem den Widerstand (2) umgebenden Teil der Ladung (5) konzentriert.

3. Miniaturventil nach Anspruch 2, **dadurch gekennzeichnet, daß** die vorgegebene elektrische Energie weniger als 10 Joule beträgt.

4. Miniaturventil nach einem der Ansprüche 1 bis 3, **dadurch gekennzeichnet, daß** die Ladung (5) und der Widerstand (2) auf derselben Seite von einem verdünnten Bereich (8) des Substrats (1) angeordnet sind, wobei die Dicke dieses Bereichs so gewählt ist, daß der Druck der Gase infolge der Verbrennung der Ladung (5) die Fragmentierung dieses Bereichs (8) und seine Öffnung für den Durchgang der Gase und anderer ggf. vorliegender Fluide bewirkt.

5. Miniaturventil nach Anspruch 4, **dadurch gekennzeichnet, daß** das Substrat (1) zwei im wesentlichen parallele Seiten (1', 1") aufweist, deren eine (1') eine zentrale Vertiefung (6) aufweist, die auf Höhe der anderen Seite (1") durch den verdünnten Bereich (8) verschlossen ist.

6. Miniaturventil nach Anspruch 5, **dadurch gekennzeichnet, daß** auf der anderen Seite (1") Metallkontakte (3, 4) für die Versorgung des Widerstands (2) von einer äußeren elektrischen Energiequelle aus gebildet sind.

7. Ventil nach einem der Ansprüche 1 bis 6, **dadurch gekennzeichnet, daß** das Substrat (1) die Form eines Chip aus einem halbleitenden Werkstoff annimmt und daß die Vertiefung (6) und der Widerstand (2) auf diesem Chip durch Mikrogravur gebildet sind.

8. Ventil nach einem der vorhergehenden Ansprüche, **dadurch gekennzeichnet, daß** die Brennstoffladung aus einem Material vom Typ Nitrocellulose oder Propergol besteht.

9. Ventil nach Anspruch 7, **dadurch gekennzeichnet, daß** das Gewicht der Brennstoffladung etwa $10^{-3}$ g beträgt.

10. Ventil nach Anspruch 4, **dadurch gekennzeichnet, daß** der verdünnte Bereich (8) des Substrats (1), der den elektrischen Widerstand (2) trägt, mindestens aus einer ersten Schicht aus Siliciumdioxid und einer auf der ersten angeordneten, zweiten Schicht aus Siliciumnitrid besteht.

11. Ventil nach Anspruch 10, **dadurch gekennzeichnet, daß** die zweite Schicht aus mit Silicium angereichertem Siliciumnitrid $SiN_x$ besteht, wobei x < 1,33 ist.

12. Ventil nach Anspruch 11, **dadurch gekennzeichnet, daß** x ungefähr gleich 1,2 ist.

13. Ventil nach Anspruch 10, **dadurch gekennzeichnet, daß** die erste Schicht und die zweite Schicht aus $SiO_2$ bzw. $Si_3N_4$ Dicken von etwa 1 µm bzw. 0,22 µm aufweisen.

14. Ventil nach Anspruch 12, **dadurch gekennzeichnet, daß** die erste Schicht und die zweite Schicht aus $SiO_2$ bzw. $SiN_{1,2}$ Dicken von etwa 0,5 µm bzw. 0,22 µm aufweisen.

15. Ventil nach Anspruch 12, **dadurch gekennzeichnet, daß** die erste Schicht und die zweite Schicht aus $SiO_2$ bzw. $SiN_{1,2}$ Dicken von etwa 1,4 µm bzw. 0,6 µm aufweisen.

16. Vorrichtung zum Füllen eines Behälters (10) mit einem Fluid, das in einem an diesem Behälter anliegenden Beutel (14) enthalten ist, **dadurch gekennzeichnet, daß** sie mindestens ein Miniaturventil $(17_i)$ nach einem der Ansprüche 1 bis 15 aufweist, das so angeordnet ist, daß eine zwischen dem Beutel (14) und dem Behälter (10) vorgesehene Fluidverbindung geschlossen wird, sowie Mittel, um die Versorgung des elektrischen Widerstands (2) des Ventils selektiv zu steuern und dadurch die Öffnung der Verbindung für das im Beutel (14) enthaltene Fluid durch den im Substrat (1) des Ventils geöffneten Durchgang durch Verbrennung der von ihm getragenen Ladung zu bewirken.

17. Vorrichtung nach Anspruch 16, **dadurch gekennzeichnet, daß** sie eine Vielzahl von Miniaturventilen $(17_i)$ aufweist, die in ebensovielen zwischen dem Beutel (14) und dem Behälter (10) vorgesehenen Fluidverbindungen angeordnet sind, wobei die Öffnung jedes von ihnen durch diese Steuermittel ausgelöst wird.

18. Vorrichtung nach einem der Ansprüche 16 und 17, **dadurch gekennzeichnet, daß** sie außerdem mindestens eine flexible Hülle (18) im Inneren des Beutels (14) aufweist, die ein Miniaturventil (16) gemäß einem der Ansprüche 1 bis 7 enthält, wobei die Steuermittel die Verbrennung der von dem Ventil (16) getragenen Brennstoffladung selektiv auslösen, so daß die Hülle (18) mit den Verbrennungsgasen aufgeblasen wird, um auf diese Weise die Leerung des Beutels (14) über das oder die in den Fluidverbindungen zwischen dem Beutel (14) und dem Behälter (10) vorgesehenen Ventile (17$_i$) zu unterstützen.

19. Vorrichtung zur transdermalen Verabreichung von Arzneimitteln, in welcher eine Wirkstofflösung anfangs in einem Beutel (14) enthalten ist und dann vor dem Beginn einer Behandlung in einen Behälter (10) übertragen wird, der dazu bestimmt ist, mit der Haut eines Patienten in Kontakt zu kommen, **dadurch gekennzeichnet, daß** die Vorrichtung gemäß einem der Ansprüche 16 bis 18 ausgebildet ist.

20. Verfahren zur Herstellung eines Ventils nach einem der Ansprüche 1 bis 15, **gekennzeichnet durch** die folgenden Schritte:

   a) man bedeckt eine im wesentlichen ebene Seite eines Substrats (1) aus halbleitendem Material mit einer Sperrschicht für eine Lösung zum Gravieren dieses Materials,
   b) man bedeckt diese Sperrschicht mit mindestens einer elektrischen Isolationsschicht, wenn die Sperrschicht nicht elektrisch isolierend ist,
   c) man bildet auf der elektrischen Isolierschicht einen elektrischen Widerstand,
   d) man höhlt das Substrat (1) von der Seite (1") des Substrats aus, die der den Widerstand (2) tragenden Seite entgegengesetzt ist, **durch** Gravur bis zu der Sperrschicht aus und
   e) man bringt über dem elektrischen Widerstand (2) eine Brennstoffladung (5) auf.

21. Verfahren nach Anspruch 20, **dadurch gekennzeichnet, daß** man zur Bildung des Substrats Silicium, zur Bildung der leitenden Schicht n-dotiertes Polysilicium und zur Bildung der elektrischen Isolationsschichten Siliciumdioxid (SiO$_2$) verwendet.

22. Verfahren nach Anspruch 20, **dadurch gekennzeichnet, daß** die Sperrschicht eine elektrisch isolierende Siliciumdioxidschicht ist, daß man die Siliciumdioxidschicht mit einer Schicht aus Siliciumnitrid (23$_1$) bedeckt und daß man den elektrischen Widerstand (24) auf dieser Schicht (23$_1$) aus Siliciumnitrid bildet.

23. Verfahren nach Anspruch 21, **dadurch gekennzeichnet, daß** man eine Schicht (23$_1$) aus stöchiometrischem Siliciumnitrid Si$_3$N$_4$ durch Abscheidung aus der Dampfphase unter Niederdruck aus Dichlorsilan und Ammoniak bei etwa 750° bildet.

24. Verfahren nach Anspruch 22, **dadurch gekenn-**

**zeichnet, daß** man eine Schicht (23$_1$) aus mit Silicium angereichertem Siliciumnitrid SiN$_x$ durch Abscheidung aus der Dampfphase bei Niederdruck aus Siliciumhydrid und Ammoniak bei etwa 750°C bildet.

FIG.:1

FIG.:2

FIG.:3

FIG.:4

FIG.:5

FIG.:6

FIG.:7

FIG.: 8A

FIG.: 8B

FIG.: 8C

FIG.:8D

FIG.:8E

FIG.:9A

$22_1$     1

$22_2$

FIG.:9B

$23_1$

$22_1$     1

$22_2$

$23_2$

FIG.:9C

24

$23_1$

$22_1$     1

$22_2$

$23_2$

FIG.:9D

24

$23_1$

$22_1$     1

$22_2$

$23_2$

FIG.:9E

24

$23_1$

$22_1$     1

$22_2$

$23_2$